# EUROPEAN PATENT SPECIFICATION

(11) **EP 1 685 250 B1**
(45) Date of publication and mention of the grant of the patent: **28.10.2009**
(21) Application number: 04798462.0
(22) Date of filing: 10.11.2004
(51) Int. Cl.: C12N 15/82

(54) **PROTEINS INVOLVED IN SIGNAL TRANSDUCTION**
PROTEINE BETEILIGT AN SIGNALTRANSDUKTION
PROTEINES IMPLIQUEES DANS LA TRANSDUCTION DE SIGNAUX

(30) Priority: 10.11.2003 GB 0326194
(43) Date of publication of application: 02.08.2006
(73) Proprietor: University of Kent, Canterbury, Kent CT2 7NZ (GB)
(72) Inventor: ROBINSON, Gary, Kevin, Canterbury Kent CT2 7AR (GB); COOK, Hannah, Kent CT3 4DX (GB)
(74) Representative: Howard, Paul Nicholas
(86) International application number: PCT/GB2004/004739
(87) International publication number: WO 2005/047514

(56) References cited:
- HENTZER MORTEN ET AL: "Pharmacological inhibition of quorum sensing for the treatment of chronic bacterial infections." JOURNAL OF CLINICAL INVESTIGATION, vol. 112, no. 9, November 2003 (2003-11), pages 1300-1307, XP002316251 ISSN: 0021-9738
- ZHU JUN ET AL: "The quorum-sensing transcriptional regulator TraR requires its cognate signaling ligand for protein folding, protease resistance, and dimerization" PROCEEDINGS OF THE NATIONAL ACADEMY OF SCIENCES OF THE UNITED STATES OF AMERICA, vol. 98, no. 4, 13 February 2001 (2001-02-13), pages 1507-1512, XP002316250 ISSN: 0027-8424
- WILLIAMS PAUL ET AL: "Quorum sensing and the population-dependent control of virulence" PHILOSOPHICAL TRANSACTIONS OF THE ROYAL SOCIETY OF LONDON B BIOLOGICAL SCIENCES, vol. 355, no. 1397, 29 May 2000 (2000-05-29), pages 667-680, XP002316249 ISSN: 0962-8436
- RAMAGE GORDON ET AL: "Inhibition of Candida albicans biofilm formation by farnesol, a quorum-sensing molecule." APPLIED AND ENVIRONMENTAL MICROBIOLOGY, vol. 68, no. 11, November 2002 (2002-11), pages 5459-5463, XP002316252 ISSN: 0099-2240

## Description

### TECHNICAL FIELD

This invention is in the field of signal transduction in bacteria and particularly relates to the modulation of quorum sensing with peptide hydrolase and peptide hydrolase inhibitors.

### BACKGROUND ART

Quorum sensing is a phenomenon that was first termed in 1994 by Fuqua et al. (J. Bacteriology, 176:269-276). However, the phenomenon of "autoinduction" in the bioluminescent organism *Photobacteria fischeri* (later to become *Vibrio fischeri*) which underpinned the development of quorum sensing research was first described in 1970 by Nealson, Platt and Hastings (J. Bacteriol. 104(1):313-22). Whilst working on the physiology of luminescence of *Photobacteria fischeri* (*Vibrio fischeri*), they noticed that there was no appreciable amount of luminescence emitted by the bacteria until the population of cells had reached a concentrated culture. This phenomenon was also noticed in conjunction with the squid *Euprymna scolopes*, where the bacteria colonise the squid's light organ to a concentration of 10¹⁰ to 10¹¹ cells/ml, causing the organ to glow. However, when present in diffuse amounts in seawater, no bioluminescence is noticed. It therefore seemed that the population of bacteria could sense its own concentration and either initiate the bioluminescent pathway or remain unlit. This mechanism which combines cell-cell communication and cell density is defined as quorum sensing.

In *V. fischeri* the system is controlled by the lux operon which consists of a number of genes including *luxI*, encoding the autoinducer synthase, and *luxR* which encodes an autoinducer-dependent activator of the luminescence genes (Sitnikov et al. 1995, Mol. Microbiol.17:801-812). The autoinducer signal molecule, which in many Gram negative bacteria is N-acylated homoserine lactone (AHL), is excreted by each cell into the surrounding media. This autoinducer is then taken up by other cells and binds the LuxR receptor protein which then activates the lux operon, causing the production of luciferase and therefore bioluminescence. Kolibachuk and Greenberg (J. Bacteriol. 1993, 175(22):7307-7312) proposed a model for the signal transduction pathway that is used in this process. Their experiments suggested that the LuxR is found on the inner surface of the bacterial cytoplasmic membrane. Further evidence of this is given in "Transcriptional Activation by LuxR" (Stevens and Greenberg, 1999, in "Cell-cell signalling in bacteria" Eds. Dunny & Winams, Fuqua, C & Greenberg, EP, 2002, Nat. Rev. Mol Cell. Biol. 3, 685-695).

The phenomenon of quorum sensing is not just limited to *V. fischeri*, but has been described in a number of Gram-negative and Gram-positive bacteria. Many of these bacteria use quorum sensing to control the production of virulence determinants thus allowing the pathogenic bacteria to avoid host defences until a sufficient population has been reached. Examples of pathogens that utilise quorum sensing include *Bacillus subtilis, Streptococcus pneumoniae, Enterococcus faecalis, Pseudomonas aeruginosa, Burkholderia cepacia, Yersinia pestis, Yersinia enterocolitica* and *Yersinia pseudotuberculosis* (Cámara et al. 2002 The Lancet Infectious Diseases 2:667-676). With the emergence of organisms that are resistant to many antibiotics such as methicillin-resistant *Staphylococcus aureus* (MRSA), quorum sensing represents a novel therapeutic target offering the opportunity to attenuate virulence, thus controlling infection by blocking cell-cell communication. *Pseudomonas aeruginosa* is also known to form biofilms which are especially important in both industry and medicine as quorum sensing is known to be important in biofilm formation (Davis DG et al., 1998, Science 280, 295-298). Therefore, formation of these biofilms may be prevented by targeting the quorum sensing mechanism.

Biofilms form when bacteria adhere to surfaces in aqueous environments and begin to excrete a slimy, glue-like substance that can anchor them to all kinds of material - such as metals, plastics, soil particles, medical implant materials, and tissue. The production of this "slimy, glue-like substance" and other virulence determinants are thought to be controlled by quorum sensing (Latifi et al. 1995, Mol. Microbiol. 17:333-344; Davis DG et al., 1998, Science 280, 295-298). A biofilm can be formed by a single bacterial species, but more often biofilms consist of many species of bacteria, as well as fungi, algae, protozoa, debris and corrosion products. Essentially, biofilms may form on any surface exposed to bacteria and some amount of water (Costerton et al. 1987, Ann. Rev. Microbiol. 41:435-464). Biofilms generally comprise bacteria that are able to produce a polymer of acidic polysaccharides. Once anchored to a surface, biofilm microorganisms carry out a variety of detrimental or beneficial reactions (by human standards), depending on the surrounding environmental conditions. They are also capable of trapping nutrients and particulates that can contribute to their enhanced development and stability. Microbial biofilms on surfaces cause billions of dollars yearly of equipment damage, product contamination, energy losses and medical infections.

Traditionally, biofilms have been treated with corrosive chemicals such as chlorine or strong alkali solutions that are harsh on plumbing systems and the environment. Treatment by biocides is not always effective due to the protective nature of the biofilm matrix polymers. It is not desirable to treat biofilms in the open environment with antibiotics. There is therefore a need for a method of inhibiting biofilms.

### DISCLOSURE OF THE INVENTION

The present invention is based on the discovery that the protein complexes involved in quorum sensing are found on the outer surface of the bacterial membrane during certain stages of growth.

In particular, it has been found that the protein LuxR is an integral part of a signal transduction complex that is found on the outer surface of the *V. fischeri* bacterial membrane. The interaction of the protein LuxR and autoinducer signal molecule (which for *V. fischeri* is an N-acylated homoserine lactone, specifically N-3-(oxohexanoyl)homoserine lactone) is therefore a target for modulation to control quorum sensing, attenuate virulence and thereby control bacterial proliferation. The finding that the protein LuxR forms part of a signal transduction complex on the outer surface of the bacterial membrane facilitates extracellular modulation of the activation of LuxR or homologue of LuxR. It therefore facilitates modulation of the activation of LuxR or a homologue of LuxR by a peptide hydrolase or peptide hydrolase inhibitor, which is unable to cross the bacterial cell membrane. Furthermore, other bacteria that use quorum sensing have similar signal transduction complexes comprising a homologue of LuxR. Two such homologues are the RhlR and LasR proteins of *P. aeruginosa*.

In a first aspect, the invention provides a method of regulating quorum sensing comprising modulating the ability of LuxR to activate transcription.

The present invention may be employed in the regulation of any bacteria employing quorum sensing. Proliferation of such bacteria involve distinct stages, namely, pre-quorate, quorate and post-quorate. For modulation, preferably the bacteria are in the pre-quorate or quorate stage, more preferably they are in the pre-quorate stage.

The activation of LuxR or a homologue of LuxR may be upregulated or downregulated. Preferably the activation of LuxR or a homologue of LuxR is downregulated and more preferably is prevented.

Homologues of LuxR are proteins that share a common evolutionary ancestor with LuxR (as described by Gray & Garey, 2001, Microbiology, 147:2379-2387) and are induced in quorum sensing. Homologues of LuxR are described in PROSITE as being members of the LuxR family of proteins (see http://us.expasy.org/cgi-bin/nicedoc.pl?PS00622). Preferably they are proteins that are found on the outer surface of bacterial membranes during the pre-quorate and quorate phases of bacterial growth and which bind a signalling molecule and are then able to activate transcription. Preferably, they are proteins that share sequence identity with LuxR. Preferably homologues of LuxR share more than 40% sequence identity with LuxR (e.g. more than 50%, 60%, 70%, 80%, 90%, 95%, 99% or more). Preferably homologues of LuxR have residues corresponding to those of LuxR. Preferably homologues of LuxR have residues corresponding to the following residues of LuxR when aligned using the Clustal alignment algorithm: TRP66, TYR70, ASP79, PRO80, GLY121, GLU187 and GLY197. Preferably the homologue of LuxR is selected from the group consisting of AhlR, AhyR, AsaR, BafR, BisR, BpsR, BviR, CarR, CepR, CerR, CinR, CsaR, CviR, EagR, EcbR, EchR, EsaR, ExpR, HalR, LasR, LuxS, Ml18752, MupR, PcoR, PhzR, PmIR, PpuR, PsmR, PsyR, RaiR, RhiR, RhIR, SdiA, SdiR, SmaR, SoIR, SpnR, SprR, SwrR, TraR, TriR, TrIR, TrnR, VanR, VsmR, Y4qH, YenR, YpeR, YpsR, YruR, YtbR and YukR.

LuxR and the homologues of LuxR use an N-acylated homoserine lactone as the signalling molecule.

It has been discovered that by treating bacteria with peptide hydrolase it is possible to downregulate quorum sensing. The action of the peptide hydrolase prevents LuxR or a homologue of LuxR from activating transcription. This is possible due to the presence of LuxR (or its homologues) on the outer membrane of bacteria. The invention therefore provides a method of downregulating the ability of LuxR (or a homologue thereof) to activate transcription comprising treating LuxR (or the homologue) with a peptide hydrolase. Preferably the peptide hydrolase used in the method of the invention is selected from group 3.4 as defined by the Nomenclature Committee of the International Union of Biochemistry (see http://www.chem.qmw.ac.uk/iubmb/enzyme/). In the case of LasR, preferred peptide hydrolases are Arg-C proteinase, Asp-N endopeptidase, BNPS Skatole, CNBr, chymotrypsin, clostripain, formic acid, glutamyl endopeptidase, iodosobenzoic acid, lysC, NTCB (2-nitro-5-thiocyanobenzoic acid), pepsin, proline-endopeptidase, proteinase K, Staphylococcal peptidase I, thermolysin and trypsin.

The downregulation of quorum sensing may be used to control bacterial proliferation. The methods of the invention may also be used to inhibit biofilms. As used herein, inhibition of a biofilm includes the prevention of biofilm formation and growth. Accordingly, the present invention provides a method of inhibiting a biofilm comprising treating a biofilm with a peptide hydrolase.

The method of the invention may be used for the inhibition of biofilms on all kinds of surfaces. Preferably the surface is selected from the list comprising, wood, glass, concrete, plastic, ceramic, porcelain and metal. The surfaces being treated may be found in an industrial context such as on a cooling tower at a power station or in a fluid pipeline, or in a domestic context such as kitchen work surfaces, baths, showers, sinks and toilets. The method is preferably used to inhibit biofilms on dentures, contact lenses, artificial valves or prosthetic implants, catheters, pacemakers and surgical pins. Accordingly, the present invention provides a method of inhibiting a biofilm on a surface comprising treating the surface with a peptide hydrolase. In a further embodiment, the peptide hydrolase may be integrated into the surface. This embodiment may be of particular use in kitchen utensils or where the surface is difficult to access, such as in pipelines.

Biofilms are produced by a number of different bacterial species. Preferably the method is used to inhibit biofilms produced by *Pseudomonas, Burkholderia, Klebsiella, Acinetobacter, Flavobacterium, Enterobacter* or *Aerobacter*.

The peptide hydrolase is preferably in the form of a composition. The composition may comprise one or more of an aqueous or non-aqueous carrier, a detergent, a surfactant, a biocide, a fungicide, an antibiotic, a pH regulator, a perfume, a dye or a colourant or a mixture thereof.

Said composition preferably comprises one or more of: an aqueous or a non-aqueous carrier, a detergent, a surfactant, a biocide, a fungicide, an antibiotic, a pH regulator, a perfume, a dye or a colourant or a mixture thereof.

As LuxR and its homologues are present on the outer surface of bacterial cells, they may be susceptible to degradation from external sources. They can also be internalised and/or degraded by the bacterial cell. It is possible to prevent this from happening by treating the bacteria with a peptide hydrolase inhibitor.

Therefore, in a second aspect, the invention provides a method of upregulating quorum sensing by treating bacteria with a peptide hydrolase inhibitor.

A number of peptide hydrolase inhibitors are known in the art. Preferably the peptide hydrolase inhibitor used in this method is selected from the group consisting of serine protease inhibitors (*e*.*g*. PMSF, Benzamide); cysteine (thiol) protease inhibitors (*e.g*. PHMB, leupeptin); aspartate (acidic) protease inhibitors (*e.g.* pepstatin, DAN) and metalloprotease inhibitors (*e.g.* EDTA, EGTA).

Treatment of bacteria with a peptide hydrolase inhibitor may be used to upregulate quorum sensing in any species of bacteria that utilises this system. Preferably quorum sensing is upregulated in bacteria selected from the list consisting of *Bacillus subtilis, Streptococcus pneumoniae, Staphylococcus aureas, Vibrio salmonicida, Aeromonas hydrophila, Burkhoderia ambifaria, Burkholderia pseudomallei, Burkholderia mallei, Burkholderia stabilis, Burkholderia vietnamiensis, Burkholderia multivorans, Escherichia coli, Serratia marcescens, Salmonella typhi, Brucella suis, Brucella melitensis, Yersinia ruckeri, Hafina alvei, Shigella f*/*exneri, Serratia liquefaciens, Enterococcus faecalis, Pseudomonas aeruginosa, Burkholderia cepacia, Pseudomonas fluorescens, Providencia stuartii, Klebsiella aerogenes, Yersinia pestis, Yersinia enterocolitica* or *Yersinia pseudotuberculosis*.

The methods of the invention may be carried out in Gram negative or Gram positive bacteria. Preferably, the methods are carried out on Gram negative bacteria.

It is also possible to insert exogenous genes into bacterial operons using common molecular biology techniques. These genes are then expressed by the bacteria upon activation and transcription of that operon. Therefore the invention also provides for a method of expressing an exogenous gene, wherein said exogenous gene has been inserted into the operon controlled by quorum sensing.

Any gene may be inserted, however preferred genes are those that encode proteins that are normally expressed at low levels or those that have a beneficial effect in the host. It is also likely that any protein produced by this method would be transported (like the LuxR protein) to the cell surface. The transport to the cell surface could occur by coupling the exogenous gene to the *luxR* gene. This would result in the production of an antigenic protein coupled to whole, or part of LuxR or a homologue of LuxR. This mechanism may therefore be used for the presentation of antigens. In particular antigens from pathogenic bacteria or viruses may be presented. Therefore genes expressing antigenic proteins are preferably used in this method. Upon expression, the antigenic protein would be presented at the bacterial surface and could induce an immune response. Therefore this method could provide an alternative method of vaccination.

### Peptide hydrolases

Peptide hydrolases are enzymes that irreversibly hydrolyse amide bonds in peptides and proteins. Peptide hydrolases are widely distributed and are involved in many different biological processes, from activation of proteins and peptides to degradation of proteins.

Peptide hydrolases have been classified by the Nomenclature Committee of the International Union of Biochemistry (see http://www.chem.qmw.ac.uk/iubmb/enzyme/) and can be found in group 3.4. There are currently 19 families in this group. The term "Family" is used to describe a group of peptide hydrolases in which each member shows an evolutionary relationship to at least one other member, either throughout the whole sequence or at least in the part of the sequence responsible for catalytic activity. The name of each Family reflects the catalytic activity type of the proteases in the Family. Thus, serine proteases belong to the S family, threonine proteases belong to the T family, aspartyl proteases belong to the A family, cysteine proteases belong to the C family and metalloproteases belong to the M family. Certain proteases have an unknown mechanism of action and belong to the "U" family. Examples of proteases include, trypsin, chymotrypsin, elastase, carboxypeptidase A and pepsin.

Peptide hydrolases (proteases) are typically small monomeric enzymes of Mᵣ 15000 to 35000. Exceptions include leucine aminopeptidase which is somewhat larger, having an Mᵣ of 324,000. Each peptide hydrolase has its own set of optimal functioning conditions and different enzymes recognise different cleavage sites.

In the present invention, bacterial cells can be treated with peptide hydrolases and these degrade proteins on the outside of bacterial cells. In particular, the peptide hydrolases will degrade LuxR or homologues of LuxR which are found on the exterior surface of the bacterial membrane. Such a degradation will result in the cell being unable to use quorum sensing, as a vital part of the signal transduction complex has been inhibited.

The Peptide Cutter tool (see http://us.expasy.org/tools/peptidecutter/peptidecutter_instructions.html) may be used to decide which particular peptide hydrolase should be use for a particular homologue of LuxR.

### Peptide hydrolase Inhibitors

As the name suggests, these are molecules that prevent the action of proteases (peptide hydrolases). Inhibition may be competitive, noncompetitive, uncompetitive or mixed and is generally reversible. The type of inhibition depends on the mode of action of the peptide hydrolase, such as if other external cofactors are required for enzyme action.

Examples of these are tissue inhibitors of metalloproteases (TIMPs) and the serine protease inhibitors known as seipins. These inhibitors are specific for particular peptide hydrolases and bind tightly to the activated enzyme to block its activity. These inhibitors are found in the human body and are secreted by cells at the margins of areas of active degradation in order to protect uninvolved matrix; they may also protect cell-surface proteins that are required for cell adhesion or migration.

Treatment with a peptide hydrolase inhibitor prevents external peptide hydrolases and bacterially produced peptide hydrolases from degrading LuxR and homologues thereof. Therefore LuxR and its homologues are available to bind signalling molecules and then become internalised and initiate transcription. This therefore interrupts the natural breakdown of the LuxR protein (and its homologues), thus upregulating quorum sensing. Furthermore signal transduction is potentiated and any associated downstream response is amplified.

As mentioned above, the following peptide hydrolase inhibitors may be used in method of the invention: serine protease inhibitors (*e.g*. PMSF, Benzamide); cysteine (thiol) protease inhibitors (*e*.*g.* PHMB, leupeptin); aspartate (acidic) protease inhibitors (*e.g*. pepstatin, DAN) and metalloprotease inhibitors (*e.g*. EDTA, EGTA).

### Compositions

Peptide hydrolases or peptide hydrolase inhibitors may be used in the present invention in the form of compositions comprising an effective amount of the peptide hydrolase or peptide hydrolase inhibitor. The term "effective amount" as used herein refers to an amount of an agent needed to treat, ameliorate, or prevent biofilm formation, or to exhibit a detectable remedial or preventative effect. For any compound, the effective dose can be estimated initially either in cell culture assays, for example using a flow cell. An "effective amount" of peptide hydrolase inhibitor is an amount of the agent needed to produce a noticeably higher level of transcription of the operon controlled by quorum sensing.

A cleaning composition for inhibiting biofilms may be in the form of the peptide hydrolase and a vehicle or carrier such as water or a non-aqueous vehicle. Aqueous vehicles or suspensions containing an effective amount of the active compound suitable to control, diminish, prevent, inhibit, detach, disperse, remove and/or clean a biofilm on a surface are preferred. The cleaning composition may also be in the form of a spray, a foam, a slurry, a dispensable liquid or a freeze-dried preparation of peptide hydrolase. The invention also includes a cleaning composition in the form of a toilet tank drop-in or a rim block.

The composition may further comprise a surfactant selected from the group consisting of anionic, nonionic, amphoteric, biological surfactants and mixtures thereof. The surfactant may be in the range of 0.1 % to 10% by weight.

Most preferably the composition may further comprise a detergent, a biocide, a fungicide, an antibiotic or a mixture thereof. The composition may also comprise a pH regulator, a perfume, a dye or a colourant.

A composition for use in upregulating transcription may contain an aqueous or a non-aqueous carrier.

### Downregulation of Gene expression

As mentioned above, peptide hydrolases may be used to disrupt quorum sensing by destroying proteins of the signal transduction complex such as LuxR and its homologues. This results in a lower level of expression than normal of the protein(s) controlled by the regulon controlled by quorum sensing. This protein could be luciferase (as is the case for *V*. *fischeri* and the Lux operon) or could be for a virulence determinant (as is the case for *Ps. aeruginosa* and the LasR operon) or could be other proteins, such as those that enable bacteria to form biofilms, for example rhamnosyltransferases RhIA and B which are under quorum sensing control by the LuxR homologue, RhIR, in *Ps. aeruginosa*.

Therefore if the expression of genes encoding virulence determinants is inhibited, it is likely to reduce the virulence of certain bacteria and also to reduce the biofilm-forming potential of bacteria.

### Upregulation of Gene Expression

The use of peptide hydrolase inhibitors results in the upregulation of quorum sensing, as the receptor complex on the outside of the bacterial cell is not degraded. It is therefore possible to insert DNA encoding desirable proteins into the Lux operon (or homologous operon) and use this system to express or over express the protein.

DNA may be inserted into bacterial cells using methods well known in the art. The DNA may be in the form of a plasmid. In order for the DNA to insert into the operon controlled by quorum sensing it is necessary to know some of the DNA sequence of this operon. This enables the design of flanking sequences that are homologous a sequence in the operon. These flanking sequences are then attached at either end of the desired exogenous gene. Once inside the bacterial cell, the desired exogenous gene with the flanking sequences may undergo homologous recombination, thus inserting the DNA of the desired gene into the operon controlled by quorum sensing. Therefore, when transcription is induced by the action of LuxR or one of its homologues, the desired gene is expressed by the bacterial cell.

This method is desirable for the production of proteins. The use of bacteria to express proteins is well known in the art. However, this method provides an easily controllable system where the production of protein can not only be switched on and off, it can be controlled by the addition of different concentrations of the signalling molecule.

As LuxR is transported to the bacterial membrane, it is likely that any exogenous protein expressed using this method would be transported to the membrane. This would be particularly useful if the secretion of proteins were desired. Alternatively, it could be useful if the proteins were retained and presented at bacterial membrane. This could provide an alternative method of vaccination. An otherwise non-pathogenic bacterium could be transformed to express an antigenic protein that could elicit an immune response. This could provide an effective means of vaccinating against viral and bacterial diseases.

### BRIEF DESCRIPTION OF DRAWINGS

Figure 1 shows the changes in bioluminescence for *V. fischeri* cells incubated under a number of conditions.
Figure 2 shows the changes in bioluminescence for *V. fischeri* cells incubated with or without peptide hydrolase inhibitor or treated with peptide hydrolase inhibitor for 15 minutes before washing and returning to fresh medium.
Figure 3 shows the changes in A) optical density and B) bioluminescence for *V. fischeri* cells incubated with or without peptide hydrolase inhibitor.

### MODES FOR CARRYING OUT THE INVENTION

### Example 1: Treatment of cells with peptide hydrolase

*Vibrio fischeri* NRRL B-11177 (ATCC 7744). The cells were grown at 25°C in 50ml volume medium in 250ml flasks in an orbital shaker set at 200rpm. Growth of cultures was measured by reading the optical density at a wavelength of 595nm using a Pye Unicam P8600 Spectrophotometer with cuvettes of 1cm light path and sterile growth medium as a blank. *Vibrio fischeri* cultures were grown in nutrient broth (Oxoid) +2% NaCl or in luminescence media consisting of 5% yeast extract, 5% tryptone peptone, 1% CaCO₃ and 3% glycerol in filtered seawater.

### Luminometry

1ml aliquots of the culture samples were placed into cylindrical, flat-bottomed cuvettes. No washing of the cells was required and light output was measured using a BioOrbit 1253 luminometer connected to a computer running the Lumicom™ data processing software. Light output was measured on a linear arbitrary scale, assuming zero to be complete darkness. Each reading was performed in triplicate and averaged.

### Peptide hydrolase Addition

Overnight cultures of *V. fischeri* were grown in NB + 2% NaCl as described previously. 1ml aliquots were taken and washed three times in Ix PBS. 10µl of a general bacterial peptide hydrolase mix (Flavourzyme from *Aspegillus oryzae*, Sigma P-6110) was added to each sample and left to incubate at room temperature for 30 minutes. Each sample was then inoculated into a 250ml flask containing 100ml sterile NB + 2% NaCl. 10µl of a 5mg/ml concentration AHL was then added to each flask and changes in optical density (595nm) and light output were monitored over time against control flasks.

### Example 2: Treatment of cells with peptide hydrolase inhibitor

The procedure was followed as for the peptide hydrolase addition experiments but with the addition of a bacterial peptide hydrolase inhibitor cocktail (Sigma, P 8465), containing 4-(2-aminoethyl)benzenesulfonyl fluoride, pepstatin A, E-64, bestatin, and sodium EDTA, in place of peptide hydrolase.

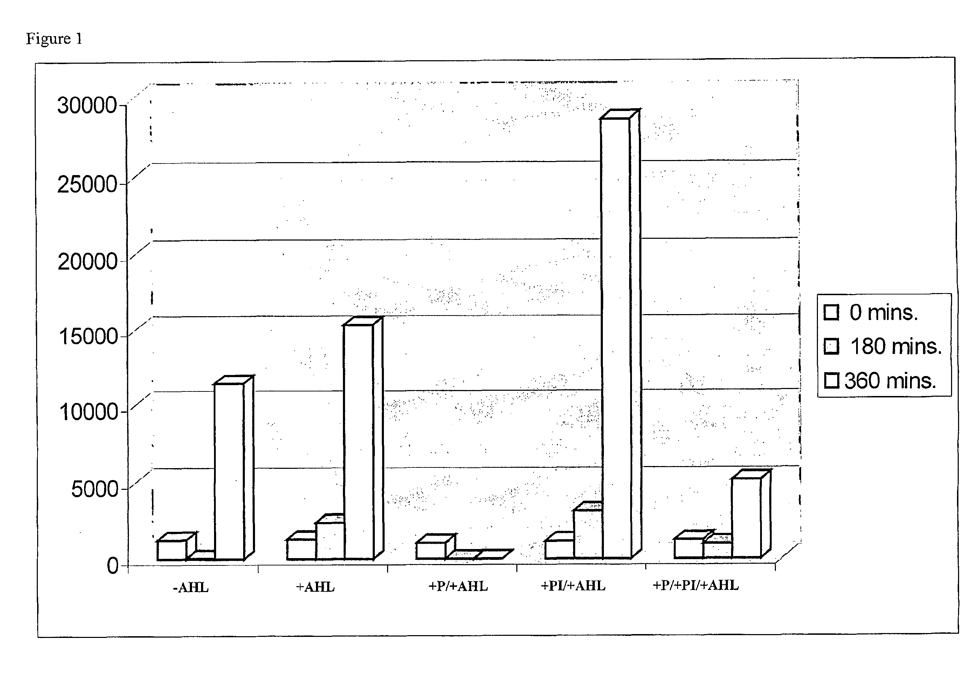

Figure 1 shows the results of the experiments conducted using both peptide hydrolase and peptide hydrolase inhibitors. As a control, luminescence was measured both with and without addition of AHL, the quorum sensing signalling molecule. The third column of figure 1 shows that upon treatment with peptide hydrolase, luminescence was completely inhibited. Conversely, when cells were treated with peptide hydrolase inhibitor rather than peptide hydrolase, the luminescence almost doubled, indicating an increase in activation of the quorum sensing system. When cells were treated with both peptide hydrolase and peptide hydrolase inhibitor a decrease in luminescence was noticed.

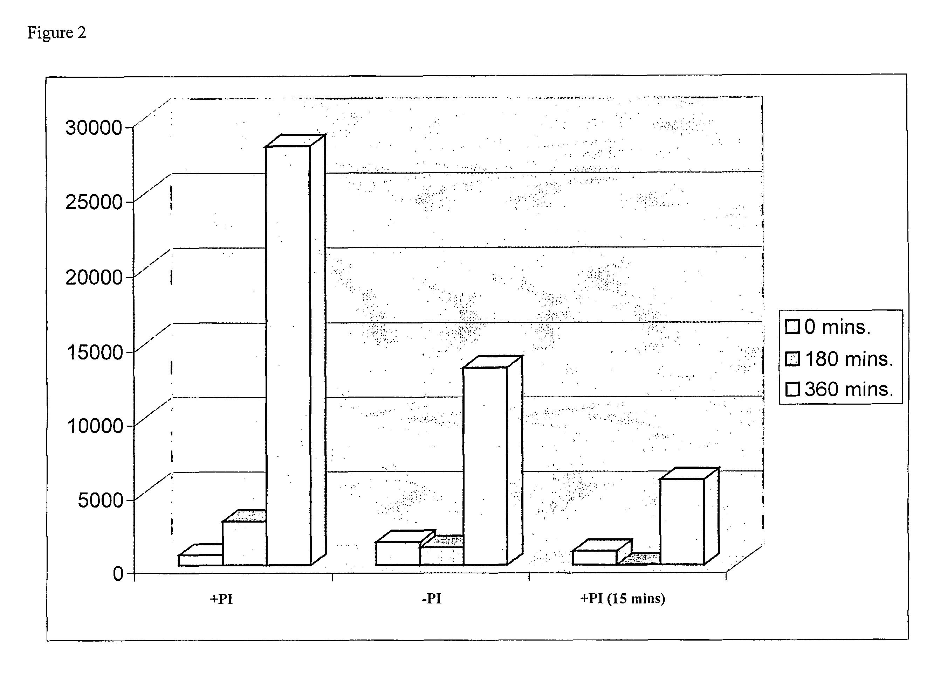

It can be seen in figure 2 that peptide hydrolase inhibitor is required in the medium in order to have its effect. When cells were treated with peptide hydrolase inhibitor, washed and then returned to fresh medium, no increase in luminescence was noted. In fact, a lower level of luminescence was noticed than for cells that had not received any peptide hydrolase inhibitor treatment.

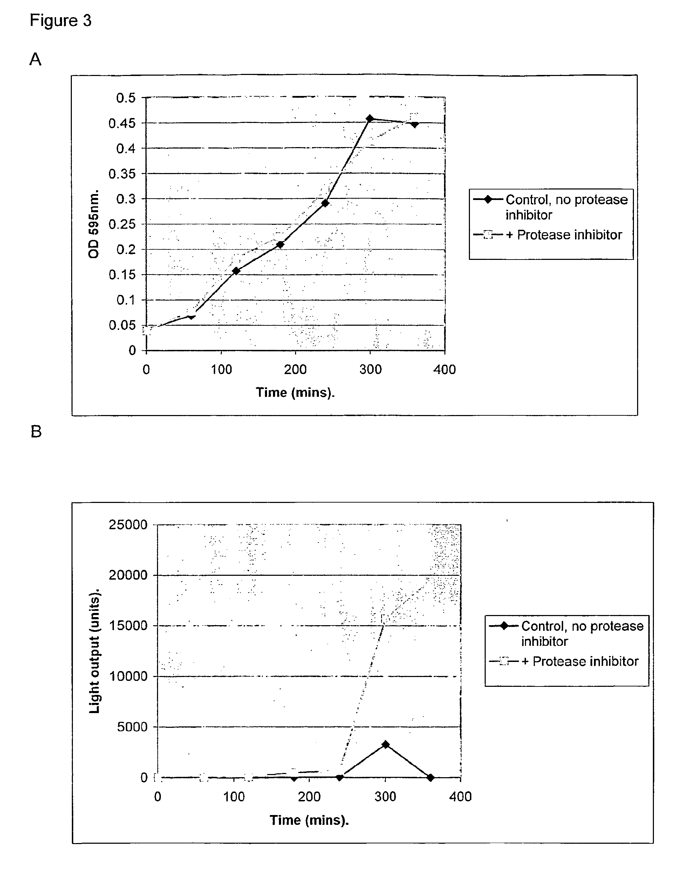

The treatment with peptide hydrolase inhibitor did not affect the growth rate of the cells as can be seen in figure 3A. However, the presence of peptide hydrolase inhibitor did produce an increase in luminescence.

## Claims

1. A method of regulating quorum sensing comprising extracellular modulation of the ability of LuxR or a homologue of LuxR to activate transcription, wherein quorum sensing is either i) downregulated by treating the bacteria with a peptide hydrolase or ii) upregulated by treating with a peptide hydrolase inhibitor.

2. A method according to claim 1 wherein said homologue of LuxR is selected from the list consisting of AhIR, AhyR, AsaR, BafR, BisR, BpsR, BviR, CarR, CepR, CerR, CinR, CsaR, CviR, EagR, EcbR, EchR, EsaR, ExpR, HaIR, LasR, Mll8752, MupR, PcoR, PhzR, PmlR, PpuR, PsmR, PsyR, RaiR, RhiR, RhlR, SdiA, SdiR, SmaR, SolR, SpnR, SprR, SwrR, TraR, TriR, TrlR, TrnR, VanR, VsmR, Y4qH, YenR, YpeR, YpsR, YruR, YtbR and YukR.

3. A method according to claim 2 wherein said peptide hydrolase is selected from the group consisting of Arg-C proteinase, Asp-N endopeptidase, BNPS Skatole, CNBr, chymotrypsin, clostripain, formic acid, glutamyl endopeptidase, iodosobenzoic acid, lysC, NTCB (2-nitro-5-thiocyanobenzoic acid), pepsin, proline-endopeptidase, proteinase K, Staphylococcal peptidase I, thermolysin and trypsin.

4. A method according to claim 3 wherein biofilm formation on a surface is inhibited.

5. A method according to claim 4 wherein said biofilm is caused by *Pseudomonas, Burkholderia, Klebsiella, Acinetobacter, Flavobacterium, Enterobacter* or *Aerobacter*.

6. A method according to claim 4 or claim 5 wherein said surface is wood, glass, concrete, plastic, ceramic, porcelain or metal.

7. A method according to any one of claims 4 to 6 wherein said surface forms part of a denture, a contact lens, an artificial valve, a prosthetic implant, a catheter, a pacemaker or a surgical pin.

8. Use of a composition comprising a peptide hydrolase and an aqueous or a non-aqueous carrier for extracellular disruption of the quorum sensing signal pathway of bacteria.

9. A use according to claim 8, wherein the composition further comprises one or more compounds selected from the group consisting of a detergent, a surfactant, a biocide, a fungicide, an antibiotic or a mixture thereof.

10. A use according to claim 8 or claim 9 wherein the composition further comprises one or more of a pH regulator, a perfume, a dye or a colorant.

11. A use according to any one of claims 8 to 10, wherein said composition is in the form of a spray, a foam, a slurry, a dispensable liquid or is freeze dried

12. A method according to claim 1 or claim 2 wherein said peptide hydrolase inhibitor is selected from the group consisting of serine protease inhibitors, including PMSF and Benzamide; cysteine (thiol) protease inhibitors, including PHMB and leupeptin; aspartate (acidic) protease inhibitors, including pepstatin and DAN; and metalloprotease inhibitors, including EDTA and EGTA.

13. A method according to claim 12 wherein said bacteria is *Vibrio salmonicida, Aeromonas hydrophila, Burkholderia ambifaria, Burkholderia pseudomallei, Burkholderia mallei, Burkholderia stabilis, Burkholderia vietnamiensis, Burkholderia multivorans, Escherichia coli, Serratia marcescens, Salmonella typhi, Brucella suis, Brucella melitensis, Yersinia ruckeri, Hafnia alvei, Shigella flexneri, Serratia liquefaciens, Enterococcus faecalis, Pseudomonas aeruginosa, Burkholderia cepacia, Pseudomonas fluorescens, Providencia stuartii, Klebsiella aerogenes, Yersinia pestis, Yersinia enterocolitica* or *Yersinia pseudotuberculosis.*

14. A method according to claim 12 or claim 13 wherein an exogenous gene is inserted into the operon controlled by quorum sensing.

15. A method according to claim 14 wherein said exogenous gene is required to be transported to the bacterial cell surface.

16. A method according to claim 14 wherein said exogenous gene encodes an antigen.

17. A method according to claim 16 wherein said antigen is of bacterial or viral origin.

18. Use of a composition comprising a peptide hydrolase inhibitor and an aqueous or a non-aqueous carrier for extracellular upregulation of the quorum sensing signal pathway of bacteria.

## Patentansprüche

1. Verfahren zum Regulieren des "Quorum sensing", umfassend die extrazelluläre Modulation der Fähigkeit von LuxR oder einem Homolog von LuxR die Transkription zu aktivieren, wobei das "Quorum sensing" entweder i) durch Behandeln der Bakterien mit einer Peptidhydrolase herabreguliert wird oder ii) durch Behandeln mit einem Peptidhydrolase-Inhibitor hochreguliert wird.

2. Verfahren nach Anspruch 1, wobei das Homolog von LuxR aus der Liste, bestehend aus AhlR, AhyR, AsaR, BafR, BisR, BpsR, BviR, CarR, CepR, CerR, CinR, CsaR, CviR, EagR, EcbR, EchR, EsaR, ExpR, HalR, LasR, Ml18752, MupR, PcoR, PhzR, PmlR, PpuR, PsmR, PsyR, RaiR, RhiR, RhlR, SdiA, SdiR, SmaR, SolR, SpnR, SprR, SwrR, TraR, TriR, TrlR, TrnR, VanR, VsmR, Y4qH, YenR, YpeR, YpsR, YruR, YtbR und YukR ausgewählt ist.

3. Verfahren nach Anspruch 2, wobei die Peptidhydrolase aus der Gruppe, bestehend aus Arg-C-Proteinase, Asp-N-Endopeptidase, BNPS-Skatol, CNBr, Chymotrypsin, Clostripain, Ameisensäure, Glutamyl-Endopeptidase, lodosobenzoesäure, lysc, NTCB (2-Nitro-5-thiocyanobenzoesäure), Pepsin, Prolin-Endopeptidase, Proteinase K, Staphylokokken-Peptidase I, Thermolysin und Trypsin ausgewählt ist.

4. Verfahren nach Anspruch 3, wobei die Bildung eines Biofilms auf einer Oberfläche gehemmt wird.

5. Verfahren nach Anspruch 4, wobei der Biofilm durch *Pseudomonas, Burkholderia, Klebsiella, Acinetobacter, Flavobacterium, Enterobacter* oder *Aerobacter* verursacht wird.

6. Verfahren nach Anspruch 4 oder Anspruch 5, wobei die Oberfläche Holz, Glas, Beton, Kunststoff, Keramik, Porzellan oder Metall ist.

7. Verfahren nach einem der Ansprüche 4 bis 6, wobei die Oberfläche einen Teil eines Gebisses, einer Kontaktlinse, einer künstlichen Klappe, eines prosthetischen lmplantats, eines Katheters, eines Schrittmachers oder eines chirurgischen Stifts bildet.

8. Verwendung einer Zusammensetzung, umfassend eine Peptidhydrolase und einen wäßrigen oder nicht-wäßrigen Träger zur extrazellulären Unterbrechung des "Quorum sensing"-Signaltransduktionswegs von Bakterien.

9. Verwendung nach Anspruch 8, wobei die Zusammensetzung weiter eine oder mehrere Verbindung(en), ausgewählt aus der Gruppe, bestehend aus einem Detergens, einem grenzflächenaktiven Mittel, einem Biozid, einem Fungizid, einem Antibiotikum oder einer Mischung davon, umfaßt.

10. Verwendung nach Anspruch 8 oder Anspruch 9, wobei die Zusammensetzung weiter eines oder mehrere von einem pH-Wert-Regulator, einem Parfum, einem Färbemittel oder einem Farbstoff umfaßt.

11. Verwendung nach einem der Ansprüche 8 bis 10, wobei die Zusammensetzung in der Form eines Sprays, eines Schaums, einer Aufschlämmung, einer verteilbaren Flüssigkeit oder gefriergetrocknet ist.

12. Verfahren nach Anspruch 1 oder Anspruch 2, wobei der Peptidhydrolase-Inhibitor aus der Gruppe, bestehend aus Serinprotease-Inhibitoren, einschließlich PMSF und Benzamid; Cystein- (Thiol-) Protease-Inhibitoren, einschließlich PHMB und Leupeptin; Aspartat- (sauren) Protease-Inhibitoren, einschließlich Pepstatin und DAN; und Metalloprotease-Inhibitoren, einschließlich EDTA und EGTA, ausgewählt ist.

13. Verfahren nach Anspruch 12, wobei das Bakterium *Vibrio satmonicida, Aeromonas hydrophila, Burkholderia ambifaria, Burkholderia pseudomallei, Burkhofderia mallei, Burkholderia stabilis, Burkholderia vietnamiensis, Burkholderia multivorans, Escherichia coli, Serratia marcescens, Salmonella typhi, Brucella suis, Brucella melitensis, Yersinia ruckeri, Hafnia alvei, Shigella flexneri, Serratia liquefaciens, Enterococcus faecalis, Pseudomonas aeruginosa, Burkholderia cepacia, Pseudomonas fluorescens, Providencia stuartii, Klebsiella aerogenes, Yersinia pestis, Yersinia enterocolitica* oder *Yersinia pseudotuberculosis* ist.

14. Verfahren nach Anspruch 12 oder Anspruch 13, wobei ein exogenes Gen in das durch das "Quorum sensing" kontrollierte Operon inseriert wird.

15. Verfahren nach Anspruch 14, wobei das exogene Gen zu der bakteriellen Zelloberfläche transportiert werden muß.

16. Verfahren nach Anspruch 14, wobei das exogene Gen ein Antigen kodiert.

17. Verfahren nach Anspruch 16, wobei das Antigen bakteriellen oder viralen Ursprungs ist.

18. Verwendung einer Zusammensetzung, umfassend einen Peptidhydrolase-Inhibitor und einen wäßrigen oder nicht-wäßrigen Träger zur extrazellulären Hochregulierung des "Quorum sensing"-Signaltransduktionswegs von Bakterien.

## Revendications

1. Procédé de régulation de la détection du quorum, comprenant la modulation extracellulaire de la faculté de LuxR ou d'un homologue de celui-ci à activer la transcription, la détection du quorum étant soit i) régulée négativement en traitant les bactéries à l'aide d'une hydrolase peptidique, soit ii) régulée positivement en les traitant à l'aide d'un inhibiteur de l'hydrolase peptidique.

2. Procédé selon la revendication 1, dans lequel ledit homologue de LuxR est choisi dans la liste constituée par AhIR, AhyR, AsaR, BafR, BisR, BpsR, BviR, CarR, CepR, CerR, CinR, CsaR, CviR, EagR, EcbR, EchR, EsaR, ExpR, HalR, LasR, Ml18752, MupR, PcoR, PhzR, PmlR, PpuR, PsmR, PsyR, RaiR, RhiR, RhIR, SdiA, SdiR, SmaR, SolR, SpnR, SprR, SwrR, TraR, TriR, TrlR, TrnR, VanR, VsmR, Y4qH, YenR, YpeR, YpsR, YruR, YtbR et YukR.

3. Procédé selon la revendication 2, dans lequel ladite hydrolase peptidique est choisie dans le groupe constitué par l'Arg-C protéinase, l'Asp-N endopeptidase, le BNPS-scatole, le CNBr, la chymotrypsine, la clostripaine, l'acide formique, la glutamyl endopeptidase, l'acide iodosobenzoïque, lysC, le NTCB (acide 2-nitro-5-thiocyanobenzoïque), la pepsine, la proline endopeptidase, la protéinase K, la peptidase I staphylococcique, la thermolysine et la trypsine.

4. Procédé selon la revendication 3, dans lequel la formation d'un biofilm sur une surface est inhibée.

5. Procédé selon la revendication 4, dans lequel ledit biofilm est provoqué par *Pseudomonas, Burkholderia, Klebsiella, Acinetobacter, Flavobacterium, Enterobacter* ou *Aerobacter*.

6. Procédé selon la revendication 4 ou la revendication 5, dans lequel ladite surface est du bois, du verre, du béton, du plastique, de la céramique, de la porcelaine ou du métal.

7. Procédé selon l'une quelconque des revendications 4 à 6, dans lequel ladite surface fait partie d'une prothèse dentaire, d'une lentille de contact, d'une prothèse valvulaire, d'un implant prothétique, d'un cathéter, d'un stimulateur cardiaque ou d'une broche chirurgicale.

8. Utilisation d'une composition comprenant une hydrolase peptidique et un véhicule aqueux ou non aqueux, pour une perturbation extracellulaire de la voie du signal de détection du quorum des bactéries.

9. Utilisation selon la revendication 8, dans laquelle la composition comprend en outre un ou plusieurs composés choisis dans le groupe constitué par un détergent, un agent tensio-actif, un biocide, un fongicide, un antibiotique ou un mélange de ceux-ci.

10. Utilisation selon la revendication 8 ou la revendication 9, dans laquelle la composition comprend en outre l'un ou plusieurs parmi un régulateur de pH, un parfum, un colorant ou une matière colorante.

11. Utilisation selon l'une quelconque des revendications 8 à 10, dans laquelle ladite composition est sous la forme d'un nuage de gouttelettes, d'une mousse, d'une suspension épaisse, d'un liquide distribuable ou est lyophilisée.

12. Procédé selon la revendication 1 ou la revendication 2, dans lequel ledit inhibiteur de l'hydrolase peptidique est choisi dans le groupe constitué par les inhibiteurs de la sérine protéase, comprenant le PMSF et le benzamide ; les inhibiteurs de la protéase à cystéine (thiol), comprenant le PHMB et la leupeptine ; les inhibiteurs de la protéase à aspartate (acide), comprenant la pepstatine et le DAN ; et les inhibiteurs des métalloprotéases, comprenant l'EDTA et l'EGTA.

13. Procédé selon la revendication 12, dans lequel lesdites bactéries sont *Vibrio salmonicida, Aeromonas hydrophila, Burkholderia ambifaria, Burkholderia pseudomallei, Burkholderia mallei, Burkholderia stabilis, Burkholderia vietnamiensis, Burkholderia multivorans, Escherichia coli, Serratia marcescens, Salmonella typhi, Brucella suis, Brucella melitensis, Yersinia ruckeri, Hafnia alvei, Shigella flexneri, Serratia liquefaciens, Enterococcus faecalis, Pseudomonas aeruginosa, Burkholderia cepacia, Pseudomonas fluorescens, Providencia stuartii, Klebsiella aerogenes, Yersinia pestis, Yersinia enterocolitica* ou *Yersinia pseudotuberculosis.*

14. Procédé selon la revendications 12 ou la revendication 13, dans lequel un gène exogène est inséré dans l'operon contrôlé par la détection du quorum.

15. Procédé selon la revendication 14, dans lequel il est nécessaire que ledit gène exogène soit transporté à la surface de la cellule bactérienne.

16. Procédé selon la revendication 14, dans lequel ledit gène exogène code pour un antigène.

17. Procédé selon la revendication 16, dans lequel ledit antigène est d'origine bactérienne ou virale.

18. Utilisation d'une composition comprenant un inhibiteur de l'hydrolase peptidique et un véhicule aqueux ou non aqueux pour la régulation positive extracellulaire de la voie du signal de détection du quorum des bactéries.
